# EUROPEAN PATENT APPLICATION

(11) **EP 0 653 206 A1**
(43) Date of publication of application: **17.05.1995**
(21) Application number: 93916167.5
(22) Date of filing: 14.07.1993
(51) Int. Cl.: A61K 31/045, A61K 31/105, A61K 31/22, A61K 31/23, A61K 31/66

(54) **ANTI-HIV AGENT**

(30) Priority: 28.07.1992 JP 201209/92; 24.12.1992 JP 344390/92
(71) Applicant: NISSUI PHARMACEUTICAL CO., LTD., Toshima-ku, Tokyo 170 (JP)
(72) Inventor: SHOJI, Shozo, Kumamoto-shi, Kumamoto 862 (JP)
(74) Representative: Wächtershäuser, Günter, Prof. Dr.
(86) International application number: JP9300981
(87) International publication number: WO9402124

(57) **Abstract**

An anti-HIV agent, for preventing and treating AIDS, containing as the active ingredient a disulfide represented by the general formula (I): R¹-S-S-R² (wherein R¹ represents C₂-C₂₀ alkenyl, and R² represents C₂-C₂₀ alkenyl or C₁-C₂₀ alkyl), or an unsaturated alcohol or its ester represented by the general formula (II): R¹-O-R³ (wherein R¹ represents C₂-C₂₀ alkenyl, and R³ represents hydrogen, C₂-C₂₀ acyl, phosphoryl or a phospholipid group). This agent has a potent growth inhibitor effect against persistently HIV-infected cells, against which the conventional anti-HIV agents have been inefficacious, thus being useful for treating AIDS.

## Description

### TECHNICAL FIELD

The present invention relates to an anti-HIV (Human Immunodeficiency Virus) agent useful for prophylaxis of and treatment for AIDS (Acquired Immune Deficiency Syndrome).

### BACKGROUND ART

AIDS is a disease caused by HIV infection, and the number of patients thereof is rapidly increasing since this disease was discovered in the United States of America in 1983. It has been known to use azidothymidine (AZT), didanosine (DDI) or the like, which is an anti-HIV agent, in treatment for such AIDS.

However, AZT is recognized to have a life-prolonging effect to a significant extent, but involves a problem that it has side effects such as headache, gastrointestinal disorders and a myelodepresant effect. Besides, both AZT and DDI exhibit a remarkable proliferation-inhibiting effect on cells primarily infected with HIV, but have no effect on cells already infected with HIV. Therefore, these agents have been said that although they have an effect to prevent an attack of AIDS-related complex and a life-prolonging effect on patients, they have no effect to suppress cells infected with HIV, namely, they are useless for basic treatment for AIDS.

It is accordingly an object of the present invention to provide an anti-HIV agent which has a marked proliferation-inhibiting effect on cells persistently infected with HIV, to say nothing of cells primarily infected with HIV and is useful for treatment for AIDS after infection.

### DISCLOSURE OF THE INVENTION

The present invention is directed to an anti-HIV agent comprising, as an active ingredient, a disulfide represented by the following general formula (1):

R¹-S-S-R² (1)

wherein R¹ means an alkenyl group having 2-20 carbon atoms, and R² denotes an alkenyl group having 2-20 carbon atoms or an alkyl group having 1-20 carbon atoms, or an unsaturated alcohol or an ester thereof represented by the following general formula (2):

R¹-O-R³ (2)

wherein R¹ means an alkenyl group having 2-20 carbon atoms, and R³ denotes a hydrogen atom, an acyl group having 2-20 carbon atoms, a phosphoric group or a phospholipid group.

The present invention is also directed to a prophylactic and remedial preparation for AIDS, comprising, as an active ingredient, a compound represented by the general formula (1) or (2).

The present invention is further directed to use of a compound represented by the general formula (1) or (2) for an anti-HIV agent or a prophylactic and remedial preparation for AIDS.

The present invention is still further directed to a method of inhibiting the proliferation of cells persistently infected with HIV, which comprises administering a compound represented by the general formula (1) or (2) to the cells persistently infected with HIV.

The compound represented by the formula (1) or (2) has an excellent proliferation-inhibiting effect on not only cells primarily infected with HIV, but also cells persistently infected with HIV and is high in safety, and so it is useful for prophylaxis of and treatment for AIDS.

### BRIEF DESCRIPTION OF THE DRAWINGS

FIG. 1 illustrates an influence of diallyl disulfide continuously administered on CEM cells.

FIG. 2 illustrates an influence of diallyl disulfide continuously administered on CEM/LAV cells.

FIG. 3 illustrates an influence of allyl acetate administered once on CEM cells.

FIG. 4 illustrates an influence of allyl acetate administered once on CEM/LAV cells.

FIG. 5 illustrates an influence of allyl alcohol administered once on CEM cells.

FIG. 6 illustrates an influence of allyl alcohol administered once on CEM/LAV cells.

FIG. 7 illustrates an influence of allyl n-butyrate administered once on CEM cells.

FIG. 8 illustrates an influence of allyl n-butyrate administered once on CEM/LAV cells.

FIG. 9 illustrates an influence of allyl n-caproate administered once on CEM cells.

FIG. 10 illustrates an influence of allyl n-caproate administered once on CEM/LAV cells.

FIG. 11 illustrates an influence of allyl n-caprylate administered once on CEM cells.

FIG. 12 illustrates an influence of allyl n-caprylate administered once on CEM/LAV cells.

FIG. 13 illustrates an influence of allyl n-caprate administered once on CEM cells.

FIG. 14 illustrates an influence of allyl n-caprate administered once on CEM/LAV cells.

### BEST MODE FOR CARRYING OUT THE INVENTION

As the alkenyl groups represented by R¹ and R² in the general formula (1) or (2), may be mentioned linear or branched alkenyl groups having 2-20 carbon atoms, for example, vinyl, allyl, 1-propenyl and 3-butenyl groups, and the like. Of these, alkenyl groups having 2-6 carbon atoms are preferred, with an allyl group being particularly preferred. As the alkyl group represented by R², may be mentioned linear or branched alkyl groups having 1-20 carbon atoms, for example, methyl, ethyl, n-propyl, n-butyl, n-pentyl and n-hexyl groups, and the like. Of these, alkyl groups having 1-6 carbon atoms are preferred. As examples of the acyl group represented by R³ and having 2-20 carbon atoms, may be mentioned acetyl, propionyl, butyryl, valeryl, caproyl, capryl, caprinoyl, lauroyl, myristoyl, palmitoyl and stearoyl groups, and the like. As the compound of the general formula (2) in which R³ is a phosphoric group, may be mentioned mono-, di- or tri- alkenyl phosphoric acid. As the compound of the general formula (2) in which R³ is a phospholipid group, may be mentioned compounds with an alkenyl group bonded to the phosphate residue of a glycerophospholipid.

Examples of the disulfide of the general formula (1) include diallyl disulfide, allyl methyl disulfide and allyl n-propyl disulfide. Of these, diallyl disulfide is particularly preferred. On the other hand, among the compounds of the general formula (2), the allyl esters of fatty acids are preferred, with the allyl esters of saturated fatty acids being particularly preferred.

Diallyl disulfide of the above disulfides (1) is a component of, for example, garlic (Allium sativum), and has been reported to have an effect to inhibit an experimental cancer in a rodent [Lee WW et al.: Cancer Research, 49, 2689-2692 (1989)]. However, there is no report about an anti-HIV effect. According to literature [John FB et al.: Cancer Research, 48, 5937-5940 (1988)], even when this diallyl disulfide is orally administered to a rat in an amount of 200 mg/kg, the rat is not killed. Besides, the compound has a high safety because it is a component of garlic.

The unsaturated alcohol or its ester (2) [hereinafter referred to as "the unsaturated compound (2)] is known compounds and can be prepared in accordance with a method known per se in the art. It is also well known to be safe.

Such disulfide (1) and unsaturated compound (2) exhibit a marked inhibitory effect on CEM/LAV cells which are known as a cell strain persistently infected with HIV as demonstrated in Examples which will be described subsequently. Although the mechanism of the anti-HIV effect thereof is not known, it is considered that they are involved in the acceleration or inhibition of a transcription and replication pathway of an HIV gene, namely, the course of genetic information connected with Tat so as to inhibit the proliferation of HIV. Further, the present inventors have observed as an experimental fact that when the number of carbon atoms of the acyl group represented by R³ in the unsaturated compound (2) increases, such a compound has a directly killing effect on HIV. Further, a partial similarity in structural formula, -CH=CHCH₂O- and -CH=CHCH₂S-, or CH₂=CH-O- and CH₂=CH-S- is recongnized in the compounds the effect of which was confirmed in Examples upon the expression of the inhibitory effect. Accordingly, there is a possibility that a compound which undergoes metabolism into a compound having such a partially similar structure after it is applied to a living body may exhibit the anti-HIV effect.

In the case where the disulfide (1) or the unsaturated compound (2) is administered as an anti-HIV agent or a prophylactic and remedial preparation for AIDS to a human, an excipient, binder, lubricant, disintegrator, coating, emulsifier, suspension, solvent, stabilizer, absorbefacient, ointment base and/or the like may be suitably added to the compound as needed, or it is formed of a liposome, thereby formulating a drug composition for oral administration, injection administration, intrarectal administration or the like in accordance with a method known per se in the art.

The preparation for the oral administration may preferably be in the form of a granule, tablet, sugar-coated tablet, capsule, soft capsule, pill, solution, emulsion, suspension or the like; the preparation for the injection administration may preferably be in the form for intravenous injection, intramuscular injection, subcutaneous injection, or drip injection or the like; and the preparation for the intrarectal administration may preferably be in the form of a suppository capsule or the like.

The dose of such a preparation varies according to administration route, the age and condition of the patient to be dosed, and the like. However, it is preferable that the dose is generally 1 mg to 10 g per day for an adult in terms of the disulfide (1) or the unsaturated compound (2). This amount of the preparation is dosed once or in portions.

### EXAMPLES

The present invention will hereinafter be described by reference to the following examples. However, this invention is not limited to these examples.

### Example 1:

### (1) Effect of diallyl disulfide continuously administered on uninfected CEM cells

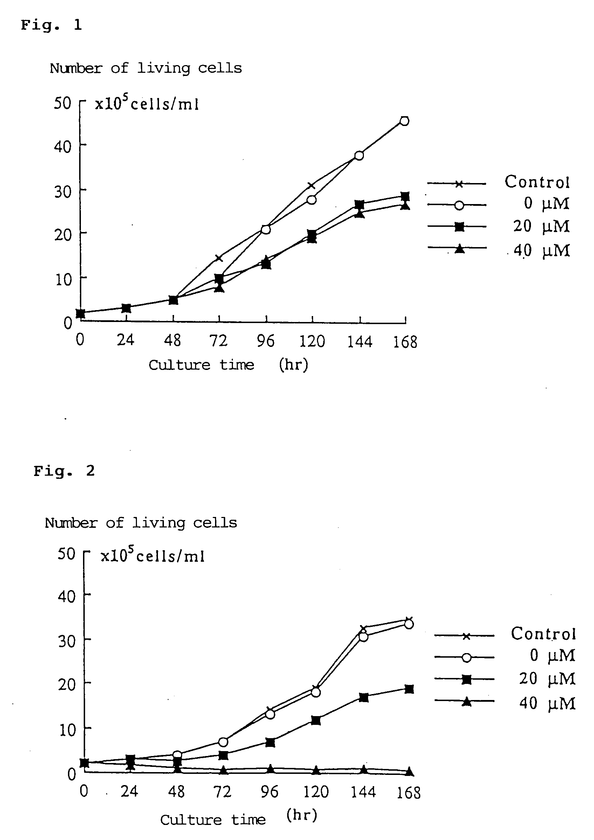

CEM cells were suspended in a culture solution RPMI-1640 (containing 10% of FCS) to give a concentration of 2.0 x 10⁵ cells/ml. This CEM cell suspension in an amount of 9.9 ml was placed in a culture bottle, to which a solution (0.01% suspension in polyoxyethylene (60) hardened castor oil) of diallyl disulfide as a test agent was added in an amount of 0.1 ml, followed by culture in an environment of 37°C and 5% CO₂. The number of living cells was counted every 24 hours by a trypan blue dye exclusion test, and at the same time, the culture was centrifuged at 1,200 rpm to collect cultured cells. A fresh culture solution and a solution of the test agent were added to the thus-collected cells to continue the culture. The result is illustrated in FIG. 1.

### (2) Effect of diallyl disulfide continuously administered on CEM/LAV cells persistently producing HIV

A suspension of CEM/LAV cells was prepared in the same manner as in the above process (1) to conduct an experiment. The result is illustrated in FIG. 2.

As apparent from FIGs. 1 and 2, diallyl disulfide in a dose of 40 µM was low in proliferation-inhibiting effect on the uninfected CEM cells, but completely inhibited the proliferation of the CEM/LAV cells persistently producing HIV. As a result, it is understood that the disulfide (1) inhibits the proliferation of cells after infected with HIV, and is useful for treatment for AIDS.

Incidentally, the influence on the CEM/LAV cells was investigated as to diallyl sulfide, diallyl ether, diethyl disulfide and di-n-propyl disulfide in the same manner as in Example 1 (2). As a result, none of these compounds exhibited a proliferation-inhibiting effect in a concentration of 20-200 µM.

### Example 2 Effect of allyl acetate:

### (1) Effect of diallyl acetate administered once on uninfected CEM cells

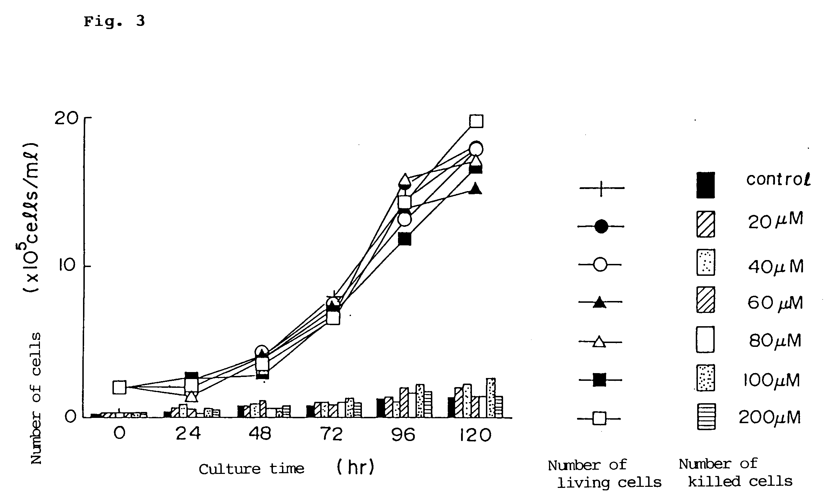

CEM cells were suspended in a culture solution RPMI-1640 (containing 10% of FCS) to give a concentration of 2.0 x 10⁵ cells/ml. This CEM cell suspension in an amount of 9.9 ml was placed in a culture bottle, to which a solution (0.01% suspension in POE (60) hardened castor oil) of diallyl acetate as a test agent was added in an amount of 0.1 ml, followed by culture in an environment of 37°C and 5% CO₂. The number of living cells was counted every 24 hours by a trypan blue dye exclusion test. The result was illustrated in FIG. 3.

### (2) Effect of diallyl acetate administered once on CEM/LAV cells persistently producing HIV

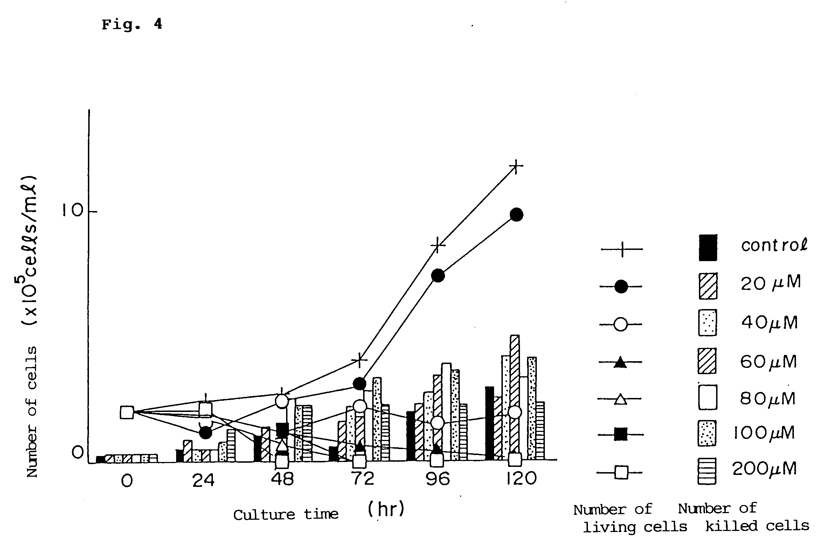

A suspension of CEM/LAV cells was prepared in the same manner as in the above process (1) to conduct an experiment. The result is illustrated in FIG. 4.

As apparent from FIGs. 3 and 4, the test agent, diallyl acetate clearly exhibited the inhibition of cell proliferation against the CEM/LAV cells persistently producing HIV, namely, increase in killed cells owing to decrease in living cells, in a concentration of 40 µM or higher. On the other hand, it did not exhibit the inhibition of cell proliferation against the uninfected CEM cells within the range of amounts used.

### Example 3 Effect of allyl alcohol:

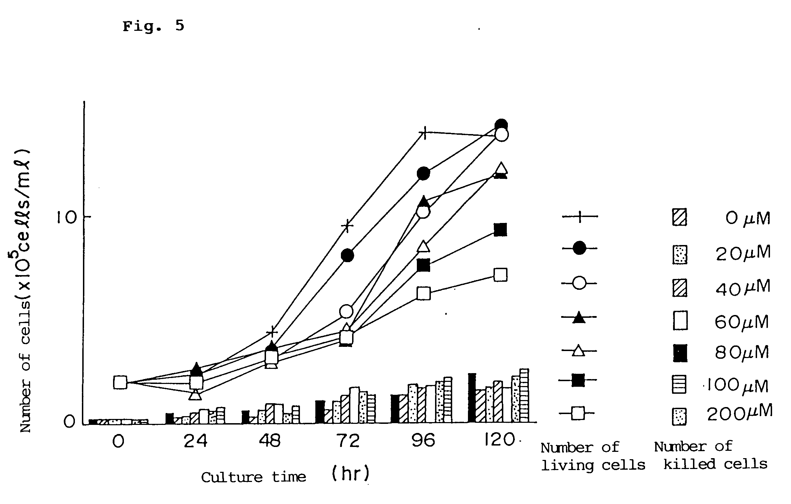

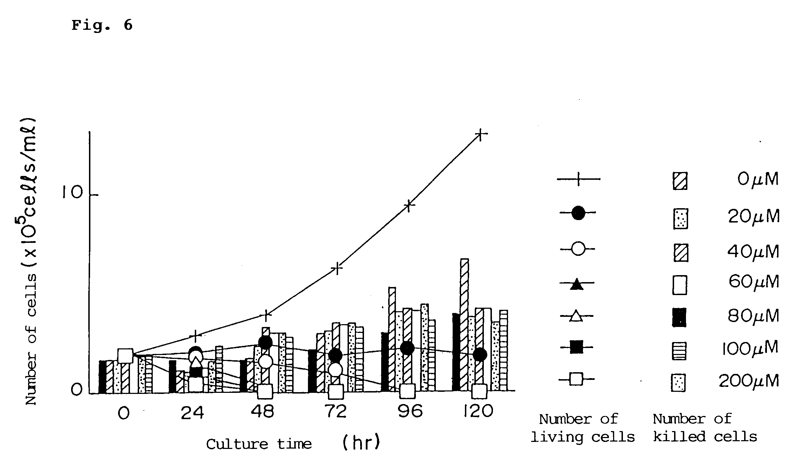

Experiments were conducted in the same manner as in Example 1 except that a test agent was changed to allyl alcohol. The results are illustrated in FIGs. 5 and 6.

As apparent from FIGs. 5 and 6, allyl alcohol exhibited a specific proliferation-inhibiting effect on the CEM/LAV cells persistently producing HIV in a concentration of 20 µM or higher.

### Example 4 Effect of allyl n-butyrate:

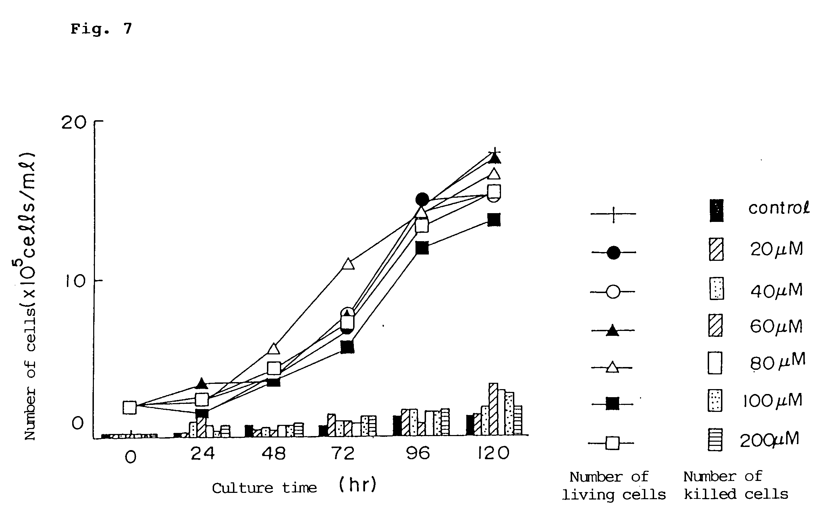

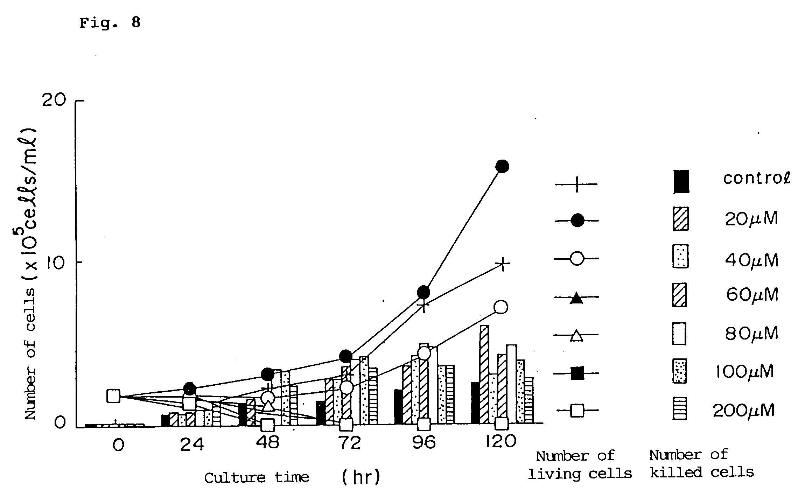

Experiments were conducted in the same manner as in Example 1 except that a test agent was changed to allyl n-butyrate. The results are illustrated in FIGs. 7 and 8.

As apparent from FIGs. 7 and 8, allyl n-butyrate exhibited a specific proliferation-inhibiting effect on the CEM/LAV cells persistently producing HIV in a concentration of 40 µM or higher.

### Example 5 Effect of allyl n-caproate:

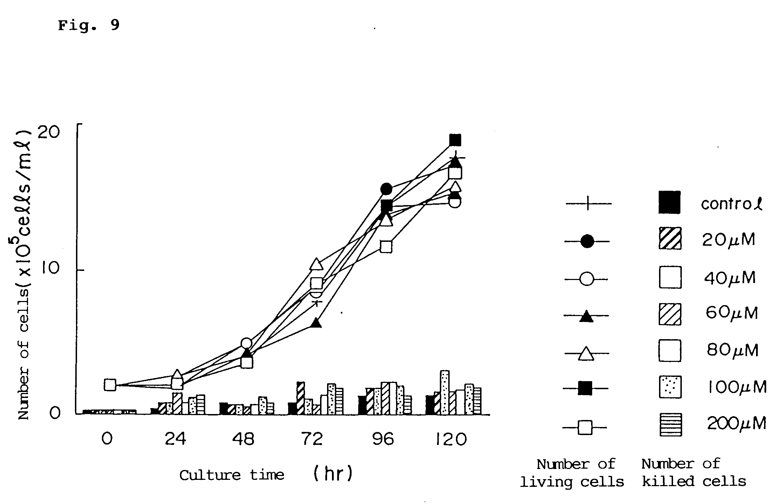

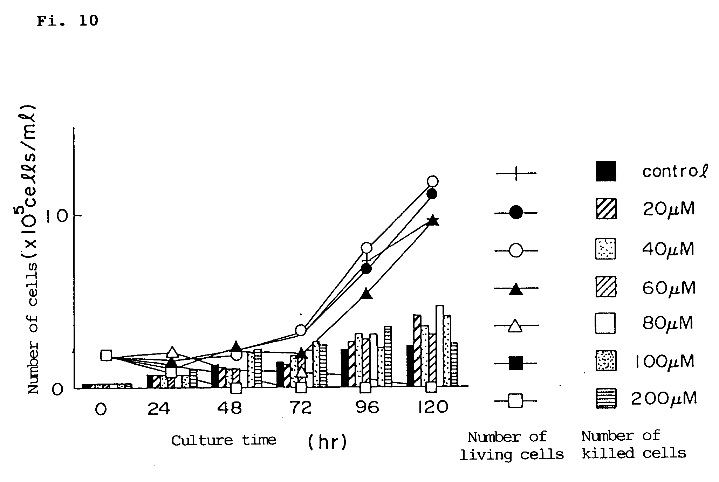

Experiments were conducted in the same manner as in Example 1 except that a test agent was changed to allyl n-caproate. The results are illustrated in FIGs. 9 and 10.

As apparent from FIGs. 9 and 10, allyl n-caproate exhibited a specific proliferation-inhibiting effect on the CEM/LAV cells persistently producing HIV in a concentration of 80 µM or higher.

### Example 6 Effect of allyl n-caprylate:

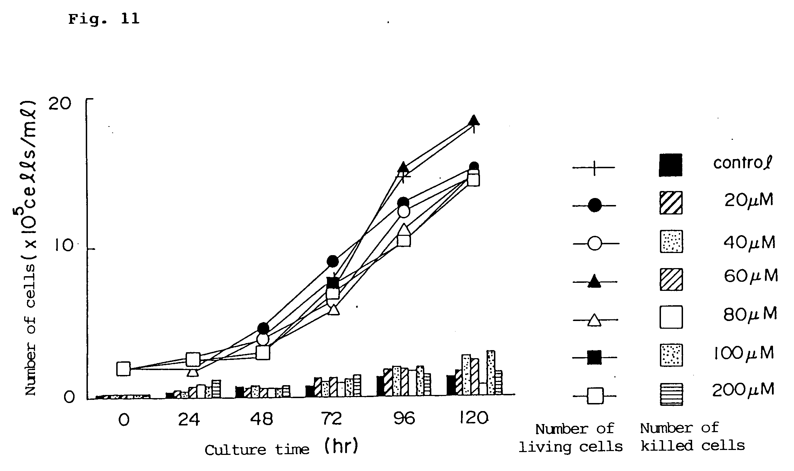

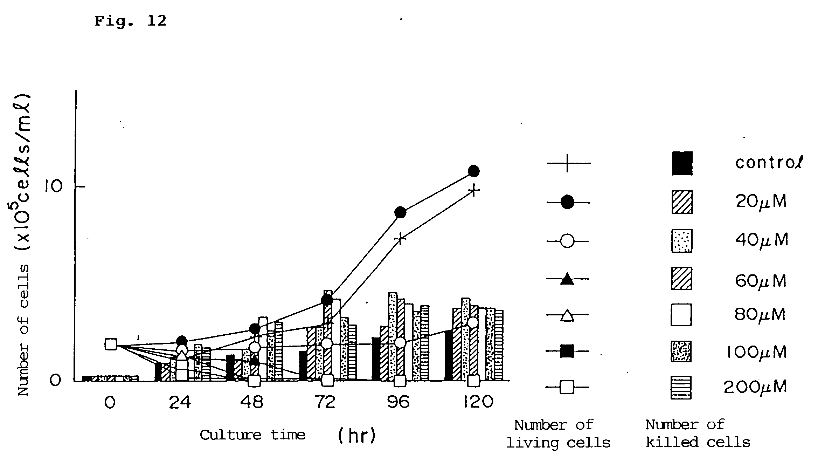

Experiments were conducted in the same manner as in Example 1 except that a test agent was changed to allyl n-caprylate. The results are illustrated in FIGs. 11 and 12.

As apparent from FIGs. 11 and 12, allyl n-caprylate exhibited a specific proliferation-inhibiting effect on the CEM/LAV cells persistently producing HIV in a concentration of 40 µM or higher.

### Example 7 Effect of allyl n-caprate:

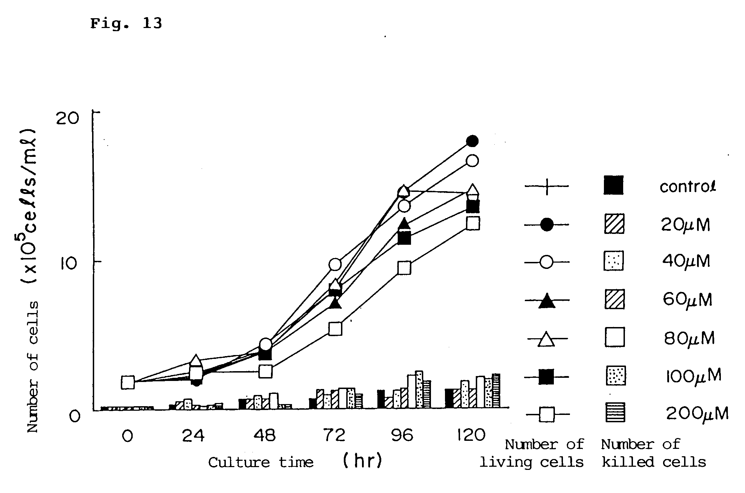

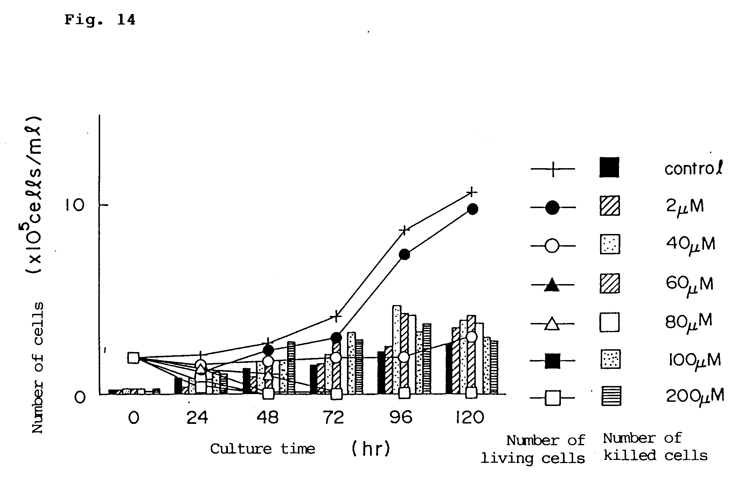

Experiments were conducted in the same manner as in Example 1 except that a test agent was changed to allyl n-caprate. The results are illustrated in FIGs. 13 and 14.

As apparent from FIGs. 13 and 14, allyl n-caprate exhibited a specific proliferation-inhibiting effect on the CEM/LAV cells persistently producing HIV in a concentration of 40 µM or higher.

### Example 8:

After the following components were granulated in accordance with a method known per se in the art, a tablet was made from the resulting granules and then coated with a film, thereby obtaining a film-coated tablet containing 20 mg of diallyl disulfide.

| | |
|---|---|
| Diallyl disulfide | 20 (mg) |
| Synthetic aluminum silicate | 60 |
| Precipitated silicic acid anhydride | 10 |
| Crystalline cellulose | Proper amount |
| Calcium carboxymethylcellulose | 40 |
| Polyvinyl pyrrolidone | 10 |
| Magnesium stearate | 5 |
| Polyvinyl acetal diethylaminoacetate | 5 |
| Hydroxypropylmethylcellulose | 5 |
| Macrogol | 3 |
| Total | 250 |

### Example 9:

The following components were mixed in accordance with a method known per se in the art and charged into a hard capsule, thereby obtaining a capsule containing 40 mg of diallyl disulfide.

| | |
|---|---|
| Diallyl disulfide | 40 (mg) |
| Synthetic aluminum silicate | Proper amount |
| Precipitated silicic acid anhydride | 20 |
| Magnesium stearate | 10 |
| Total | 200 |

### Example 10:

After the following components were granulated in accordance with a method known per se in the art, a tablet was made from the resulting granules and then coated with a film, thereby obtaining a film-coated tablet containing 20 mg of diallyl acetate.

| | |
|---|---|
| Diallyl acetate | 20 (mg) |
| Synthetic aluminum silicate | 60 |
| Precipitated silicic acid anhydride | 10 |
| Crystalline cellulose | Proper amount |
| Calcium carboxymethylcellulose | 40 |
| Polyvinyl pyrrolidone | 10 |
| Magnesium stearate | 5 |
| Polyvinyl acetal diethylaminoacetate | 5 |
| Hydroxypropylmethylcellulose | 5 |
| Macrogol | 3 |
| Total | 250 |

### Example 11:

The following components were mixed in accordance with a method known per se in the art and charged into a hard capsule, thereby obtaining a capsule containing 40 mg of diallyl acetate.

| | |
|---|---|
| Diallyl acetate | 40 (mg) |
| Synthetic aluminum silicate | Proper amount |
| Precipitated silicic acid anhydride | 20 |
| Magnesium stearate | 10 |
| Total | 200 |

### INDUSTRIAL APPLICABILITY

The disulfides (1) and the unsaturated compounds (2) have a marked proliferation-inhibiting effect on not only cells primarily infected with HIV, but also cells persistently infected with HIV, and is useful for treatment for AIDS.

## Claims

1. An anti-HIV agent comprising, as an active ingredient, a disulfide represented by the general formula (1):
R¹-S-S-R² (1)
wherein R¹ means an alkenyl group having 2-20 carbon atoms, and R² denotes an alkenyl group having 2-20 carbon atoms or an alkyl group having 1-20 carbon atoms, or an unsaturated alcohol or an ester thereof represented by the general formula (2):
R¹-O-R³ (2)
wherein R¹ means an alkenyl group having 2-20 carbon atoms, and R³ denotes a hydrogen atom, an acyl group having 2-20 carbon atoms, a phosphoric group or a phospholipid group.

2. The anti-HIV agent according to Claim 1, wherein the active ingredient is diallyl disulfide or the allyl ester of a fatty acid.

3. A prophylactic and remedial preparation for AIDS, comprising, as an active ingredient, a disulfide represented by the general formula (1):
R¹-S-S-R² (1)
wherein R¹ means an alkenyl group having 2-20 carbon atoms, and R² denotes an alkenyl group having 2-20 carbon atoms or an alkyl group having 1-20 carbon atoms, or an unsaturated alcohol or an ester thereof represented by the general formula (2):
R¹-O-R³ (2)
wherein R¹ means an alkenyl group having 2-20 carbon atoms, and R³ denotes a hydrogen atom, an acyl group having 2-20 carbon atoms, a phosphoric group or a phospholipid group.

4. The prophylactic and remedial preparation for AIDS according to Claim 3, wherein the active ingredient is diallyl disulfide or the allyl ester of a fatty acid.

5. Use of a disulfide represented by the general formula (1):
R¹-S-S-R² (1)
wherein R¹ means an alkenyl group having 2-20 carbon atoms, and R² denotes an alkenyl group having 2-20 carbon atoms or an alkyl group having 1-20 carbon atoms, or an unsaturated alcohol or an ester thereof represented by the general formula (2):
R¹-O-R³ (2)
wherein R¹ means an alkenyl group having 2-20 carbon atoms, and R³ denotes a hydrogen atom, an acyl group having 2-20 carbon atoms, a phosphoric group or a phospholipid group, for an anti-HIV agent.

6. The use of the compound for the anti-HIV agent according to Claim 5, wherein the active ingredient is diallyl disulfide or the allyl ester of a fatty acid.

7. Use of a disulfide represented by the general formula (1):
R¹-S-S-R² (1)
wherein R¹ means an alkenyl group having 2-20 carbon atoms, and R² denotes an alkenyl group having 2-20 carbon atoms or an alkyl group having 1-20 carbon atoms, or an unsaturated alcohol or an ester thereof represented by the general formula (2):
R¹-O-R³ (2)
wherein R¹ means an alkenyl group having 2-20 carbon atoms, and R³ denotes a hydrogen atom, an acyl group having 2-20 carbon atoms, a phosphoric group or a phospholipid group, for a prophylactic and remedial preparation for AIDS.

8. The use of the compound for the prophylactic and remedial preparation for AIDS according to Claim 7, wherein the active ingredient is diallyl disulfide or the allyl ester of a fatty acid.

9. A method of inhibiting the proliferation of cells persistently infected with HIV, which comprises administering a disulfide represented by the general formula (1):
R¹-S-S-R² (1)
wherein R¹ means an alkenyl group having 2-20 carbon atoms, and R² denotes an alkenyl group having 2-20 carbon atoms or an alkyl group having 1-20 carbon atoms, or an unsaturated alcohol or an ester thereof represented by the general formula (2):
R¹-O-R³ (2)
wherein R¹ means an alkenyl group having 2-20 carbon atoms, and R³ denotes a hydrogen atom, an acyl group having 2-20 carbon atoms, a phosphoric group or a phospholipid group, to the cells persistently infected with HIV.

10. The method of inhibiting the proliferation according to Claim 9, wherein the compound to be administered is diallyl disulfide or the allyl ester of a fatty acid.
